# EUROPEAN PATENT SPECIFICATION

(11) **EP 3 297 622 B1**
(45) Date of publication and mention of the grant of the patent: **01.11.2023**
(21) Application number: 16796016.0
(22) Date of filing: 17.05.2016
(51) Int. Cl.: A61K 31/164, A61K 31/4468, A61K 31/485, A61P 25/04

(54) **COMBINATIONS OF OXYCODONE AND N-ACYLETHANOLAMINE PALMITOYLETHANOLAMINE FOR REDUCING OPIOID-ASSOCIATED SIDE EFFECTS**
KOMBINATIONEN VON OXYCODON UND N-ACYLETHANOLAMIN PALMITOYLETHANOLAMIN ZUR REDUZIERUNG VON OPIOID-ASSOZIIERTEN NEBENWIRKUNGEN
COMBINAISONS D'OXYCODONE ET DE N-ACYLÉTHANOLAMINE PALMITOYLÉTHANOLAMINE POUR LA RÉDUCTION DES EFFECTS SECONDAIRES ASSOCIÉS AUX OPIOÏDES

(30) Priority: 21.05.2015 US 201562164618 P
(43) Date of publication of application: 28.03.2018
(73) Proprietor: Scisparc Ltd, Tel Aviv (IL)
(72) Inventor: SHMULEWITZ, Ascher, 6908158 Tel Aviv (IL); HABER, Elran, Kiryat Ono, 5545049 (IL); BRENER, Ephraim, 75241 Rishon LeZion (IL)
(74) Representative: Finnegan Europe LLP
(86) International application number: PCT/IL2016/050519
(87) International publication number: WO 2016/185468

(56) References cited:
- EP-A1- 2 944 309
- US-B2- 8 487 007
- GATTI A ET AL: "Palmitoylethanolamide in the treatment of chronic pain caused by different etiopathogenesis", PAIN MEDICINE, BLACKWELL SCIENCE, MALDEN, US, vol. 13, no. 9, 1 September 2012 (2012-09-01), pages 1121-1130, XP002730844, ISSN: 1526-2375, DOI: 10.1111/J.1526-4637.2012.01432.X [retrieved on 2012-07-30]
- GATTI A; SABATO E; DI PAOLO A R; MAMMUCARI M; SABATO A F: "A Low-dose synergic combination useful in different types of pain: A New Hypothesis", CLINICAL DRUG INVESTIGATION, vol. 30, no. Suppl 2, 2010, pages 3-14, XP009509494,
- DI CESARE MANNELLI ET AL.: 'Delay of morphine tolerance by palmitoylethanolamide.' BIOMED RESEARCH INTERNATIONAL 2015; vol. 2015, 2015, XP055331120
- DESIO, P.: 'Combination of oxycodone and palmitoylethanolamide for low back pain treatment.' RIVISTA SIARED DI ANESTESIA E MEDICINA CRITICA vol. 1.2, 31 December 2011, pages 62 - 71.
- JAN M. KEPPEL HESSELINK. ET AL.: 'New Targets in Pain, Non-Neuronal Cells, and the Role of Palmitoylethanolamide' THE OPEN PAIN JOURNAL vol. 5, 29 February 2012, pages 12 - 23., XP055331127

## Description

### FIELD OF THE INVENTION

The present invention relates to compositions for reducing side-effects of oxycodone or a salt thereof, wherein the side effects are selected from irritation, confusion, uncontrolled movement and/or disorientation. The present invention provides pharmaceutical compositions comprising oxycodone or a salt thereof and N-acylethanolamines for their use in a variety of indications amenable to prevention and treatment by opioids, and in preventing and ameliorating oxycodone-related side effects selected from irritation, confusion, uncontrolled movement and/or disorientation.

### BACKGROUND OF THE INVENTION

An opioid is any chemical such as morphine that resembles opiates in its pharmacological effects. Opiates are natural alkaloids found in the resin of the Papaver somniferum (opium poppy), while opioid refers to both opiates and synthetic substances, as well as to opioid peptides. Opioids work by binding to opioid receptors, which are found principally in the central and peripheral nervous system and the gastrointestinal tract (Lesniak et al., Acta. Neurobiol. Exp., 2011, Vol. 71, pages 129-138). Opioid receptors are a group of inhibitory G protein-coupled receptors, acting on γ-aminobutyric acid GABAergic neuro-transmission, with opioids as ligands. A GABAergic agent (or drug) is a chemical which functions to directly modulate the GABA system in the body or brain. Examples include GABA receptor agonists, GABA receptor antagonists, and GABA reuptake inhibitors. The endogenous opioids are dynorphins, enkephalins, endorphins, endomorphins and nociceptin. The receptors in these organ systems mediate the beneficial effects as well as the psychoactive and the side effects of opioids.

There are three principal classes of opioid receptors, µ (mu, MOR), κ (kappa, KOR) and δ (delta, DOR), although several more have been reported, including ε (epsilon), 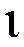 (iota), λ (lambda) and ζ (zeta) receptors. The pharmacodynamic response to an opioid depends upon the receptor to which it binds, its affinity for that receptor, and whether the opioid is an agonist or an antagonist. For example, the supraspinal analgesic properties of morphine (an opioid agonist analgesic drug) are mediated by activation of the µ1 receptor; respiratory depression and physical dependence by the µ2 receptor; and sedation and spinal analgesia by the κ receptor.

Each group of opioid receptors elicits a distinct set of neurological responses, with the receptor subtypes (such as µ1 and µ2 for example) providing even more specific responses. Unique to each opioid is its distinct binding affinity to the various classes of opioid receptors (e.g. the µ, κ, and δ opioid receptors are activated at different magnitudes according to the specific receptor binding affinities of the opioid).

The µ receptors which bind morphine and its derivatives are responsible for analgesia, respiratory and gastrointestinal functions, sedation, neuro-endocrine functions and mediate opiate dependence. The δ receptors are abundant in the central nervous system (CNS) and mediate analgesia, feeding and various hormonal functions. The κ receptors have a wide distribution in the CNS and the peripheral nervous system (PNS); for example, centrally, these receptors are expressed in caudate-putamen, nucleus accumbens, amygdale, neural lobe of the pituitary gland, etc, and peripherally, they are expressed in the sensory neuron posterior root ganglion (DRG), stomach, duodenum, jejunum, oleum, proximal and distal colon (Lesniak and Lipkowski, Acta. Neurobiol. Exp., 2011, Vol. 71(1), pages 129-138). The κ receptors are responsible for functions including analgesia, gastrointestinal functions like food intake, gut motility, water balance, thermoregulation and various neuroendocrine functions (Leander et al., J. Pharmacol. Exp. Ther., 1985, Vol. 234(2), pages 463-469; Goodman and Gilman's The Pharmacological Basis of Therapeutics (12th Edition), 2011, Chapter 18, pages 460-501). US Patent application 2015/0031685 describes substituted heterocyclic acetamides as kappa opioid receptor (KOR) agonists, and claims their use in treating e.g. gastrointestinal dysfunction, ileus and pain. US Patent application 2015/0072971 describes mu opioid receptor (MOR) agonists, and relates to their use in treating depressive symptoms such as depressed mood, loss of pleasure, loss of appetite, sleep disturbance, psychomotor changes, fatigue, and post-partum depression.

Opioids generally act on specified opioid receptor, for example morphine acts on µ-opioid receptor, whereas oxycodone mainly binds κ-opioid receptor. However, most of the opioids will bind different opioid receptors with various degree of affinity. Moreover, certain opioids act as opioid-receptor agonists and as opioid-receptor antagonists on different opioid receptors.

Most clinically used opioid analgesics, such as morphine and codeine, act as µ receptor agonists. These opioids have well-known, undesirable and potentially dangerous dependence-forming side effects associated with their activity in CNS. Another widely used opioid is oxycodone, which acts on x-opioid receptors and appears to be a κ2b-opioid agonist.

Opioids are effective and widely used medicines for pain management in cancer patients and non-cancer patients. The analgesic (pain-killer) effects of opioids are due to decreased perception of pain, decreased reaction to pain, and/or increased pain tolerance. However, use of opioids often has debilitating adverse effects such as psychological addiction, tolerance, opioid-induced dysfunction, including respiratory depression, esophageal dysfunction, endocrine disruption, and opioid-induced bowel dysfunction (OIBD), which comprises opioid-induced constipation, dry mouth, nausea, vomiting, gastric stasis, bloating, and abdominal pain (Benyamin et al., Pain Physician, 2008, Vol. 11(2 Suppl), pages S105-S120).

N-acylethanolamines (NAEs) are lipid-derived signaling molecules. They are formed when one of several types of acyl group is linked to the nitrogen atom of ethanolamine. Examples of *N*-acylethanolamines include anandamide (the amide of arachidonic acid (20:4 ω-6) and ethanolamine), N-Palmitoylethanolamine (the amide of palmitic acid (16:0) and ethanolamine), N-Oleoylethanolamine (the amide of oleic acid (18:1) and ethanolamine), N-Stearoylethanolamine (the amide of stearic acid (18:0) and ethanolamine) and N-Docosahexaenoylethanolamine (the amide of docosahexaenoic acid (22:6) and ethanolamine).

Palmitoylethanolamide (PEA, also known as N-(2-hydroxyethyl)hexadecanamide; Hydroxyethylpalmitamide; palmidrol; N-palmitoylethanolamine; and palmitylethanolamide) is an endogenous fatty acid amide, belonging to the class of nuclear factor agonists. PEA has been demonstrated to bind to a receptor in the cell nucleus (a nuclear receptor) and exerts a variety of biological functions related to chronic pain and inflammation. Studies have shown that PEA interacts with distinct non-CB 1/CB2 receptors, suggesting that PEA utilizes a unique "parallel" endoopioid signaling system. This concept was further supported by growing evidences that PEA production and inactivation can occur independently of AEA and 2-AG production and inactivation. Much of the biological effects of PEA on cells can be attributed to its affinity to PPAR (particularly PPAR-α and PPAR-γ). PEA was shown to have an affinity to opioid-like G-coupled receptors GPR55 and GPR119 as well as the transient receptor potential vanilloid type 1 receptor (TRPV1). PEA has been shown to have anti-inflammatory, anti-nociceptive, neuro-protective, and anti-convulsant properties.

Described by S. Ben-Shabat et al. (Eur. J. Pharmacol., 1998, Vol. 353(1), pages 23-31), the concept of the "entourage effect" is that plant opioids act together, or possess synergy, and affect the body in a mechanism similar to the body's own endo-opioid system. This theory serves as the foundation for a somewhat controversial idea within pharmacology, that in certain cases whole plant extractions serve as better therapeutic agents than individual opioid extractions. The "entourage effect" theory has been expanded in recent times by Wagner and Ulrich-Merzenich (Phytomedicine, 2009, Vol. 16(2-3), pages 97-110), who define the four basic mechanisms of whole plant extract synergy as follows: (a) ability to affect multiple targets within the body, (b) ability to improve the absorption of active ingredients, (c) ability to overcome bacterial defense mechanisms, and (d) ability to minimize adverse side-effects.

A number of clinical trials have described combinations of PEA and other analgesics, e.g. opiates and antiepileptic drugs used for neuropathic pain (reviewed in J.M.K. Hesselink, The Open Pain Journal, 2012, Vol. 5, pages 12-23). For example, Desio and coworkers (Rivista Siared di Anestesia e Medicina Critica, 2011, Vol. 1(2), pages 62-71) conducted an open study in patients suffering from chronic pain and unresponsive to a variety of analgesics. Patients were daily treated with 5 mg oxycodone for 5 days, followed by 10 mg oxycodone for 25 days, in combination (via separate administrations) with 1200 mg PEA.

Further, Di Paolo and coworkers (34th National Congress AISD - New frontiers in pain medicine, 2011, Riccione, Italy) reported a study in which patients suffering from chronic pain were daily treated with 1200 mg PEA for 21 days, followed by 600 mg PEA for a further 30 days, in combination (via separate administrations) with oxycodone hydrochloride.

In addition, Mannelli and coworkers (BioMed Research International, 2015, article ID 894732) recently reported the results of a study in which rats were daily injected with 30 mg/kg PEA (subcutaneously) and 10 mg/kg morphine (intraperitoneally).

Further limitations and disadvantages of conventional and traditional approaches will become apparent to one of skill in the art, though comparison of such systems with some aspects of the present invention as set forth in the remainder of the present application with reference to the drawings.

There remains a need in the field of therapy by opioids for pharmaceutical combinations of opioids and other agents capable *e.g.* of increasing the potency of opioids in humans. A need also exists for compositions and methods to prevent, postpone, treat and/or minimize the side effects of opioids and opioid dependence.

### SUMMARY OF THE INVENTION

The present invention provides pharmaceutical compositions comprising beneficial combinations of opioids and N-acylethanolamines. as defined in the claims These combinations are defined, in part, by specific molar ratios between the respective active agents and/or by their dosages, and may be employed in a variety of methods. Further, the present invention provides compositions for use in methods for preventing and/or treating conditions or side-effects associated with opioid uptake, such as irritation or confusion.

The provision of such combinations provides great benefits over other compositions and methods utilizing opioids alone. For example, the compositions provided herein potentiate the therapeutic effect of prescribed opioids, which may be clinically translated to a more beneficial therapeutic result or to the use of lower dosages of opioids to obtain a predetermined therapeutic result. The compositions provided herein advantageously eliminate or substantially minimize adverse side-effects commonly associated with opioid uptake in opioid-prescribed patients. In other words, according to the principles of the present invention, the therapeutic window (or pharmaceutical window) of the opioid, *i.e.* the range of opioid dosages which can be prescribed and/or treat conditions effectively without having toxic effects, is expended by the combinations provided herein.

The provision of the combinations provided herein also has great benefits over other compositions and methods utilizing opioids and N-acylethanolamines administered separately. For example, as the N-acylethanolamines are mixed with and cannot be separated from the opioids, the compositions provided herein eliminate the risks involved in consuming opioids alone, such as irritation, confusion, and formation of opioid dependence and addiction, as exemplified herein. In addition, patients treated with opioids alone tend to develop tolerance to the drug, which leads to increasing dosage of the drug being needed for exerting the analgesic effect and to subsequent withdrawal symptoms. In general, opioid drugs are given by prescription, but once prescribed and if they are given orally or transdermally, they are self-administered (ex. Oxycodone, Fentanil, Tramadol, Hydromorphine). Furthermore, codeine may be purchased even without the prescription. Abuse and diversion of opioids is a growing problem as the availability of these medications increases and this public health issue undermines their clinical utility. As summarized by R. Benyamin and coworkers (Pain Physician, 2008, Opioid Special Issue, Vol. 11, pages S105-S120), it is estimated that the prevalence of a substance abuse disorder is 8% in the general population and even higher in the population of patients with chronic pain, which reflects a dramatic rise in prescription drug abuse, including one study which showed that 16% of active pain patients were found to be abusing their medications. Thus, without being restricted to any theory or mechanism, the proviso of a single composition for both opioids and N-acylethanolamines serves as a safety measure against accidental and/or deliberate intake of opioids alone.

The present invention is based, in part, on the surprising findings that combinations of opioids and N-acylethanolamines were able to prevent or ameliorate a variety of side-effects associated with intake of opioids in an *in-vivo* model, and that these combinations were further able to increase the effect of opioids as analgesic agents. Without being bound to any theory or mechanism, it is hypothesized that administration of opioids in mixture with N-acylethanolamines increases the potency of opioids while decreasing their related side-effects, a phenomenon previously labeled "entourage effect".

The present invention provides, in one aspect, a pharmaceutical composition for use in a method for preventing or treating at least one side-effect associated with intake of an opioid, wherein the opioid is oxycodone or a salt thereof, wherein the at least one side-effect is selected from irritation, confusion, uncontrolled movement and/or disorientation, wherein the composition comprises a therapeutically-effective amount of a mixture of at least one opioid or a salt thereof and at least one N-acylethanolamine or a salt thereof, wherein the molar ratio between the opioid or the salt thereof and the N-acylethanolamine or the salt thereof is between about 1:1 to about 1:100, wherein the at least one opioid is oxycodone or a salt thereof, and wherein the N-acylethanolamine is palmitoylethanolamine (PEA) or a salt thereof. In certain embodiments, the molar ratio is between about 1:2 to about 1:80. In certain embodiments, the molar ratio is between about 1:2.5 to about 1:5.

In certain embodiments, the pharmaceutical composition described above comprises about 1-100 mg opioid or a salt thereof. In certain embodiments, the pharmaceutical composition described above comprises about 20 mg, about 35 mg, about 55 mg, about 70 mg or about 90 mg opioid or a salt thereof. The at least one opioid is oxycodone or a salt thereof.

In certain embodiments, the pharmaceutical composition described above comprises about 200-1800 mg N-acylethanolamine or a salt thereof. In certain embodiments, the pharmaceutical composition described above comprises about 250 mg, about 500 mg, about 750 mg, about 1000 mg or about 1500 mg N-acylethanolamine or a salt thereof. The N-acylethanolamine is PEA or a salt thereof.

The mixture comprises oxycodone or a salt thereof and PEA or a salt thereof. In certain embodiments, the molar ratio between the oxycodone or the salt thereof and the PEA or the salt thereof is between about 1:2.5 to about 1:5. In certain embodiments, the molar ratio between the oxycodone or the salt thereof and the PEA or the salt thereof is about 1:2.5. In certain embodiments, the molar ratio between the oxycodone or the salt thereof and the PEA or the salt thereof is about 1:5. In certain embodiments, the mixture comprises about 1-100 mg oxycodone or a salt thereof or about 200-1800 mg PEA or a salt thereof. In certain embodiments, the mixture comprises about 20 mg, about 35 mg, about 55 mg, about 70 mg or about 90 mg oxycodone or a salt thereof or about 250 mg, about 500 mg, about 750 mg, about 1000 mg or about 1500 mg PEA or a salt thereof. In certain embodiments, the mixture comprises about 1-100 mg oxycodone or a salt thereof and about 200-1800 mg PEA or a salt thereof. In certain embodiments, the mixture comprises about 20 mg, about 35 mg, about 55 mg, about 70 mg or about 90 mg oxycodone or a salt thereof and about 250 mg, about 500 mg, about 750 mg, about 1000 mg or about 1500 mg PEA or a salt thereof. Each possibility represents a separate embodiment of the invention.

In certain embodiments, the pharmaceutical composition described above is formulated for systemic administration. In certain embodiments, the pharmaceutical composition described above is formulated for oral, oral mucosal, nasal, sublingual, topical, transdermal, rectal, parenteral, intravenous, intramuscular, subcutaneous, intrathecal, inhalational or vaginal administration. In certain embodiments, the pharmaceutical composition described above is formulated for oral, oral mucosal, nasal, or sublingual administration. Each possibility represents a separate embodiment of the invention.

The present invention further provides, in another aspect, a dosage unit comprising or consisting of any one of the pharmaceutical compositions described above.

The present invention provides a pharmaceutical composition described above, for use in a method for preventing or treating at least one side-effect associated with intake of an opioid.

In certain embodiments, the side-effect is irritation. In certain embodiments, the side-effect is confusion, uncontrolled movement and/or disorientation. Each possibility represents a separate embodiment of the invention. In certain embodiments, the side-effect is confusion.

In certain embodiments, the side-effect is associated with intake of oxycodone or a salt thereof.

As disclosed herein, the therapeutic potency of the pharmaceutical composition is increased compared to the therapeutic potency of the same pharmaceutical composition without the N-acylethanolamine. As disclosed herein, the required therapeutic dosage of the opioid in the pharmaceutical composition is decreased compared to the required therapeutic dosage of the opioid of the same pharmaceutical composition without the N-acylethanolamine. In certain embodiments, at least one of the side-effects of intake of at least one opioid is ameliorated compared to the same side-effect of intake of the same opioid of the same pharmaceutical composition without the N-acylethanolamine. As disclosed herein, the therapeutic window of the opioid is expended compared to the therapeutic window of the opioid of the same pharmaceutical composition without the N-acylethanolamine. As disclosed herein, the therapeutic effect of the pharmaceutical composition decreases slower than the therapeutic effect of the same pharmaceutical composition without the N-acylethanolamine.

The present invention further provides, in another aspect, a composition for use in a method for preventing or treating at least one side-effect associated with intake of opioid, wherein the opioid is oxycodone or a salt thereof, in a human subj ect in need thereof, the method comprising the step of administering to the subject a pharmaceutical composition comprising a therapeutically-effective amount of a mixture of at least one opioid or a salt thereof and at least one N-acylethanolamine or a salt thereof, wherein the molar ratio between the opioid and the N-acylethanolamine is between about 1:1 to about 1:100, thereby preventing or treating the at least one side-effect.

In certain embodiments, the administration step is repeated.

Further embodiments and the full scope of applicability of the present invention will become apparent from the detailed description given hereinafter.

### BRIEF DESCRIPTION OF THE DRAWINGS

Figures 1A and 1B are bar graphs illustrating different group averages of body weight gain during the study.
**Figure 2** is a bar graph illustrating different group averages of animal velocity during evaluation in an open field test.
**Figure 3** is a bar graph illustrating different group averages of total time spent in the center of an arena during evaluation in an open field test.
Figures 4A and 4B are bar graphs illustrating different group averages of latency to respond in a tail pinch test.

### DETAILED DESCRIPTION OF THE INVENTION

The present invention provides pharmaceutical combinations comprising at least one opioid and at least one N-acylethanolamine for use in preventing opioid-related side-effects. For example, the compositions of the invention can be used to treat pain. The compositions of the invention can also be used to induce and maintain anesthesia, and as an analgesic for pain of various etiologies.

Besides pain, conditions such as cough, diarrhea, anxiety, and shortness of breath are also routinely treated with opioids. Opioid therapy is frequently accompanied by opioid-related side effects, which include, but are not limited to, addiction, which involves a compulsive use of a drug, constipation, drowsiness, nausea and vomiting, diarrhea, muscle and bone pain, insomnia, anxiety, cold flashes with goose bumps ("cold turkey"), involuntary leg movements and irritability. When abused, even a single large dose of opioid can cause severe respiratory depression and death. Thus, there is a great need to decrease the dosages of opioids in treatment, reduce their associated side effects and/or increase the safety of opioid administration/intake.

The pharmaceutical compositions of the invention provide an improved medicament: exhibiting an increased opioid therapeutic activity, minimizing administered opioid dosages, prolonging opioid therapeutic window and reducing the risk of developing opioid-related side-effects. In other words, the present invention discloses that N-acylethanolamine compounds exhibit an opioid-sparing effect. The term "opioid-sparing" as used herein refers to the enablement of the use of low dosages of opioids in instances wherein a mid- or high-dosages of opioids are typically required. The opioid and N-acylethanolamine compounds according to the present invention include pharmaceutically acceptable forms thereof, including isomers such as diastereomers and enantiomers, salts, solvates, and polymorphs, as well as racemic mixtures.

The pharmaceutical combinations provided by the present invention, in which opioids are mixed and combined with N-acylethanolamines, eliminate this inherent, adverse dichotomy between therapeutic efficacy and tolerability by potentiating the therapeutic effect of prescribed opioids, which is clinically translated to a more beneficial therapeutic result or to the use of lower dosages of opioids to obtain a predetermined therapeutic result. The pharmaceutical combinations provided by the present invention advantageously eliminate or substantially minimize adverse side-effects commonly associated with opioid uptake in opioid-prescribed patients. According to the principles of the present invention, the therapeutic window of the opioids is expended by the pharmaceutical combinations provided herein such that (a) standard opioid dosages, e.g. in the range of 10-90 mg oxycodone daily, are better tolerated due to reduced side-effects, and (b) standard opioid dosages can be markedly reduced without compromising their therapeutic efficacy or the patient's health due to the increased efficacy of the opioid.

The present invention provides, in one aspect, a pharmaceutical composition for use according to claim 1.

The molar ratio between the opioid or the salt thereof and the N-acylethanolamine or the salt thereof is between about 1:1 to about 1:100.

The present invention provides a pharmaceutical composition for use according to claim 1 comprising a therapeutically-effective amount of a mixture of at least one opioid or a salt thereof and at least one N-acylethanolamine or a salt thereof, wherein the molar ratio between the opioid or the salt thereof and the N-acylethanolamine or the salt thereof is between about 1:1 to about 1:100.

As used herein, a "pharmaceutical composition" refers to a preparation of the active agents described herein with other chemical components such as physiologically suitable carriers and excipients. The purpose of a pharmaceutical composition is to facilitate administration of a compound to an organism. As used herein, the phrase "pharmaceutically acceptable carrier" refers to a carrier, an excipient or a diluent that does not cause significant irritation to an organism and does not abrogate the biological activity and properties of the administered compound. An adjuvant is included under these phrases.

The term "excipient" as used herein refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, oils such as vegetable oils or fish oils, and polyethylene glycols.

The term "carrier" as used herein refers to a diluent, adjuvant, excipient, or vehicle with which the compound is administered. Such pharmaceutical carriers can be sterile liquids, such as water and oils. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin, 18th Edition.

The phrase "pharmaceutically acceptable" as used herein refers to molecular entities and compositions that are physiologically tolerable and do not typically produce an allergic or similar toxicity when administered to an individual. Preferably, and particularly where a formulation is used in humans, the term "pharmaceutically acceptable" may mean approved by a regulatory agency (for example, the U.S. Food and Drug Agency) or listed in a generally recognized pharmacopeia for use in animals (e.g., the U.S. Pharmacopeia).

The term "opioid" generally refers to a compound that binds to opioid receptors. As used herein, the term "opioid" also encompasses all natural, semi-synthetic and synthetic opioids. In particular, opioid include drugs acting on opioid receptors present in the central nervous system and/or peripheral system, as well as those acting on opioid receptors present in the gastrointestinal tract. Examples of natural opioids include, but are not limited to, morphine, codeine, thebaine, dihydrocodein, and salvinorin A. Examples of semi-synthetic opioids include, but are not limited to, opium alkaloids derivatives such as diacetylmorphine, hydrocodone, dihydrocodeine, hydromorphone, oxymorphone, desomorphine, nicomorphine, oxycodone, dipropanoylmorphine, benzylmorphine and ethylmorphine. Examples of fully synthetic opioids include, but are not limited to, methadone, tramadol, propoxyphene anilidopiperidines (e.g., fentanyl), phenylpiperidines (e.g., pethidine), diphenylpropylamine derivatives (e.g., loperamide), benzomorphan derivatives (e.g., dezocine), oripavine derivatives (e.g., buprenorphine), and morphinan derivatives (e.g., butorphanol). It also includes endogenous opioid peptides, which may be produced naturally in the body as endorphins, dynorphins or enkephalins but which can also be synthesized.

The term "N-acylethanolamine" as used herein generally refers to a type of fatty acid amide, lipid-derived signaling molecules, formed when one of several types of acyl group is linked to the nitrogen atom of ethanolamine. These amides conceptually can be formed from a fatty acid and ethanolamine with the release of a molecule of water, but the known biological synthesis uses a specific phospholipase D to cleave the phospholipid unit from N-acylphosphatidylethanolamines. The suffixes -amine and -amide in these names each refer to the single nitrogen atom of ethanolamine that links the compound together: it is termed "amine" in ethanolamine because it is considered as a free terminal nitrogen in that subunit, while it is termed "amide" when it is considered in association with the adjacent carbonyl group of the acyl subunit. Names for these compounds may be encountered with either "amide" or "amine" in the present application. The term "ethanolamine" is used in the generic sense and is meant to include mono-ethanolamine, di-ethanolamine, tri-ethanolamine, and mixtures thereof.

The term "derivative" as used herein means a compound whose core structure is the same as, or closely resembles that of an N-acylethanolamine compound, but which has a chemical or physical modification, such as different or additional side groups.

The term "salt" as used herein refers to any form of an active ingredient in which the active ingredient assumes an ionic form and is coupled to a counter ion (a cation or anion) or is in solution. This also includes complexes of the active ingredient with other molecules and ions, in particular complexes which are complexed by ion interaction.

In certain embodiments, the molar ratio is between about 1:1 to about 1:90, about 1:1 to about 1:80, about 1:1 to about 1:70, about 1:1 to about 1:60, about 1:1 to about 1:50, about 1:1 to about 1:40, about 1:1 to about 1:30, about 1:1 to about 1:20 or about 1:1 to about 1:10. In certain embodiments, the molar ratio is between about 1:2 to about 1:80, about 1:2 to about 1:70, about 1:2 to about 1:60, about 1:2 to about 1:50, about 1:2 to about 1:40, about 1:2 to about 1:30, about 1:2 to about 1:20 or about 1:2 to about 1:10. In certain embodiments, the molar ratio is between about 1:2 to about 1:80. In certain embodiments, the molar ratio is between about 1:2.5 to about 1:5. Each possibility represents a separate embodiment of the present invention. In certain embodiments, the molar ratio is about 1:2.5. In certain embodiments, the molar ratio is about 1:5. In certain embodiments, the molar ratio is at least about 1:2.5. In certain embodiments, the molar ratio is at least about 1:5.

In certain embodiments, the pharmaceutical composition described above comprises about 1-100 mg opioid or a salt thereof. In certain embodiments, the pharmaceutical composition described above comprises about 5-90 mg, about 10-80 mg, about 20-70 mg, about 30-60 mg or about 40-50 mg opioid or a salt thereof. Each possibility represents a separate embodiment of the present invention.

In certain embodiments, the pharmaceutical composition described above comprises about 1-90 mg, about 1-80 mg, about 1-70 mg, about 1-60 mg, about 1-50 mg, about 1-40 mg, about 1-30 mg, about 1-20 mg or about 1-10 mg opioid or a salt thereof. In certain embodiments, the pharmaceutical composition described above comprises about 2-100 mg, about 5-100 mg, about 10-100 mg, about 20-100 mg, about 30-100 mg, about 40-100 mg, about 50-100 mg, about 60-100 mg, about 70-100 mg, about 80-100 mg or about 90-100 mg opioid or a salt thereof. Each possibility represents a separate embodiment of the present invention.

In certain embodiments, the pharmaceutical composition described above comprises about 10 mg, about 20 mg, about 30 mg, about 40 mg, about 50 mg, about 60 mg, about 70 mg, about 80 mg or about 90 mg opioid or a salt thereof. In certain embodiments, the pharmaceutical composition described above comprises at least about 10 mg, at least about 20 mg, at least about 30 mg, at least about 40 mg, at least about 50 mg, at least about 60 mg, at least about 70 mg, at least about 80 mg or at least about 90 mg opioid or a salt thereof. In certain embodiments, the pharmaceutical composition described above comprises less than about 10 mg, less than about 20 mg, less than about 30 mg, less than about 40 mg, less than about 50 mg, less than about 60 mg, less than about 70 mg, less than about 80 mg or less than about 90 mg opioid or a salt thereof. Each possibility represents a separate embodiment of the present invention.

In certain embodiments, the pharmaceutical composition described above comprises about 20 mg opioid or a salt thereof. In certain embodiments, the pharmaceutical composition described above comprises 35 mg opioid or a salt thereof. In certain embodiments, the pharmaceutical composition described above comprises about 55 mg opioid or a salt thereof. In certain embodiments, the pharmaceutical composition described above comprises about 70 mg opioid or a salt thereof. In certain embodiments, the pharmaceutical composition described above comprises about 90 mg opioid or a salt thereof.

The at least one opioid is oxycodone or a salt thereof. In certain embodiments, the at least one opioid is oxycodone. In certain embodiments, the at least one opioid is a salt of oxycodone. In certain embodiments, the at least one opioid consists of oxycodone or a salt thereof. In certain embodiments, the at least one opioid consists of oxycodone.

In certain embodiments, the pharmaceutical composition comprises about 200-1800 mg N-acylethanolamine or a salt thereof. In certain embodiments, the pharmaceutical composition comprises about 250-1550 mg, about 300-1200 mg, about 350-950 mg, about 400-700 mg, about 450-600 mg or about 500-550 mg N-acylethanolamine or a salt thereof. Each possibility represents a separate embodiment of the present invention. In certain embodiments, the pharmaceutical composition comprises at least about 50 mg, at least about 100 mg, at least about 150 mg, at least about 200 mg, at least about 250 mg, at least about 300 mg, at least about 350 mg, at least about 400, at least about 450 mg, at least about 500 mg, at least about 550 mg, at least about 600 mg, at least about 650 mg, at least about 700 mg, at least about 750 mg, at least about 800 mg, at least about 850 mg, at least about 900 mg, at least about 950 mg, at least about 1000 mg, at least about 1050 mg, at least about 1100 mg, at least about 1150 mg, at least about 1200 mg, at least about 1250 mg, at least about 1300 mg, at least about 1350 mg, at least about 1400 mg, at least about 1450 mg, at least about 1500 mg, at least about 1550 mg, at least about 1600 mg, at least about 1650 mg, at least about 1700 mg, at least about 1750 mg or at least about 1800 mg N-acylethanolamine or a salt thereof. In certain embodiments, the pharmaceutical composition comprises about 50 mg, about 100 mg, about 150 mg, about 200 mg, about 250 mg, about 300 mg, about 350 mg, about 400, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg or about 1800 mg N-acylethanolamine or a salt thereof. Each possibility represents a separate embodiment of the present invention.

The N-acylethanolamine is PEA or a salt thereof. In certain embodiments, the N-acylethanolamine consists of PEA or a salt thereof. In certain embodiments, the N-acylethanolamine consists of PEA.

The mixture comprises oxycodone or a salt thereof and PEA or a salt thereof. In certain embodiments, the mixture consists of oxycodone or a salt thereof and PEA or a salt thereof. In certain embodiments, the mixture comprises oxycodone and PEA. In certain embodiments, the mixture consists of oxycodone and PEA. In certain embodiments, the molar ratio between the oxycodone or the salt thereof and the PEA or the salt thereof is between about 1:2.5 to about 1:5. In certain embodiments, the molar ratio between the oxycodone or the salt thereof and the PEA or the salt thereof is about 1:2.5. In certain embodiments, the molar ratio between the oxycodone or the salt thereof and the PEA or the salt thereof is about 1:5. In certain embodiments, the mixture comprises about 1-100 mg oxycodone or a salt thereof or about 200-1800 mg PEA or a salt thereof. In certain embodiments, the mixture comprises about 20 mg, about 35 mg, about 55 mg, about 70 mg or about 90 mg oxycodone or a salt thereof or about 250 mg, about 500 mg, about 750 mg, about 1000 mg or about 1500 mg PEA or a salt thereof. In certain embodiments, the mixture comprises about 1-100 mg oxycodone or a salt thereof and about 200-1800 mg PEA or a salt thereof.

In certain embodiments, the mixture comprises about 5-90 mg, about 10-80 mg, about 20-70 mg, about 30-60 mg or about 40-50 mg oxycodone or a salt thereof and about 250-1550 mg, about 300-1200 mg, about 350-950 mg, about 400-700 mg, about 450-600 mg or about 500-550 mg PEA or a salt thereof.

In certain embodiments, the mixture comprises about 10 mg, about 20 mg, about 30 mg, about 40 mg, about 50 mg, about 60 mg, about 70 mg, about 80 mg or about 90 mg oxycodone or a salt thereof and at least about 50 mg, at least about 100 mg, at least about 150 mg, at least about 200 mg, at least about 250 mg, at least about 300 mg, at least about 350 mg, at least about 400, at least about 450 mg, at least about 500 mg, at least about 550 mg, at least about 600 mg, at least about 650 mg, at least about 700 mg, at least about 750 mg, at least about 800 mg, at least about 850 mg, at least about 900 mg, at least about 950 mg, at least about 1000 mg, at least about 1050 mg, at least about 1100 mg, at least about 1150 mg, at least about 1200 mg, at least about 1250 mg, at least about 1300 mg, at least about 1350 mg, at least about 1400 mg, at least about 1450 mg, at least about 1500 mg, at least about 1550 mg, at least about 1600 mg, at least about 1650 mg, at least about 1700 mg, at least about 1750 mg or at least about 1800 mg PEA or a salt thereof.

In certain embodiments, the mixture comprises about 10 mg, about 20 mg, about 30 mg, about 40 mg, about 50 mg, about 60 mg, about 70 mg, about 80 mg or about 90 mg oxycodone or a salt thereof and about 50 mg, about 100 mg, about 150 mg, about 200 mg, about 250 mg, about 300 mg, about 350 mg, about 400, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg or about 1800 mg PEA or a salt thereof.

In certain embodiments, the mixture comprises about 20 mg, about 35 mg, about 55 mg, about 70 mg or about 90 mg oxycodone or a salt thereof and about 250 mg, about 500 mg, about 750 mg, about 1000 mg or about 1500 mg PEA or a salt thereof. Each possibility represents a separate embodiment of the invention.

In certain embodiments, the pharmaceutical composition described above is formulated for systemic administration. In certain embodiments, the pharmaceutical composition described above is formulated for oral, oral mucosal, nasal, sublingual, topical, transdermal, rectal, parenteral, intravenous, intramuscular, subcutaneous, intrathecal, inhalational or vaginal administration. In certain embodiments, the pharmaceutical composition described above is formulated for oral, oral mucosal, nasal, or sublingual administration. Each possibility represents a separate embodiment of the invention.

Techniques for formulation and administration of drugs are well known in the art, and may be found, e.g. in "Remington's Pharmaceutical Sciences," Mack Publishing Co., Easton, Pa. Pharmaceutical compositions of the present invention may be manufactured by processes well known in the art, e.g., by means of conventional mixing, dissolving, granulating, dragee-making, levigating, emulsifying, encapsulating, entrapping, or lyophilizing processes.

For oral administration, the pharmaceutical composition can be formulated by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries as desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, and sodium carbomethylcellulose; and/or physiologically acceptable polymers such as polyvinylpyrrolidone (PVP). If desired, disintegrating agents, such as cross-linked polyvinyl pyrrolidone, agar, or alginic acid or a salt thereof, such as sodium alginate, may be added.

The term "oral administration" refers to any method of administration in which an active agent can be administered by swallowing, chewing, sucking, or drinking an oral dosage form. Examples of solid dosage forms include conventional tablets, multi-layer tablets, capsules, caplets, etc., which do not substantially release the drug in the mouth or in the oral cavity.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions, and suitable organic solvents or solvent mixtures. Dyestuffs or pigments may be added to the tablets or dragee coatings for identification or to characterize different combinations of active compound doses.

Pharmaceutical compositions that can be used orally include stiff or soft, sealed capsules made of gelatin and a plasticizer, such as glycerol or sorbitol. The capsules may contain the active ingredients in admixture with filler such as lactose, binders such as starches, lubricants such as talc or magnesium stearate, and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration. For buccal and sublingual administration, the compositions may take the form of tablets or lozenges formulated in conventional manner or in adhesive carriers. Alternatively, the active ingredient may be in powder form for constitution with a suitable vehicle, e.g., a sterile, pyrogen-free, water-based solution, before use.

Pharmaceutical compositions suitable for use in the context of the present invention include compositions wherein the active ingredients are contained in an amount effective to achieve the intended purpose. More specifically, a "therapeutically effective amount" means an amount of active ingredients effective to prevent, alleviate, or ameliorate symptoms or side effects of a disease or disorder, or prolong the survival of the subject being treated. Determination of a therapeutically effective amount is well within the capability of those skilled in the art, especially in light of the detailed disclosure provided herein. More specifically, a "therapeutically effective amount of a mixture" means an amount of at least two active ingredients, wherein each one of the active ingredients independently may not be in a therapeutically effective amount or wherein both of the active ingredients may not be in a therapeutically effective amount, the mixture is nevertheless effective to prevent, alleviate, or ameliorate symptoms or side effects of a disease or disorder, or prolong the survival of the subject being treated. The term "mixture" as used herein refers to a non-covalent combination of two molecules.

For any preparation used in the compositions of the invention, the dosage or the therapeutically effective amount can be estimated initially from in-vitro, in-vivo and cell culture assays. For example, a dose can be formulated in animal models to achieve a desired concentration or titer. Such information can be used to more accurately determine useful doses in humans. The dosage of each compound of the claimed combinations depends on several factors, including: the administration method, the disease to be treated, the severity of the disease, whether the disease is to be treated or prevented, and the age, weight, and health of the person to be treated. Additionally, pharmaco-genomic (the effect of genotype on the pharmacokinetic, pharmaco-dynamic or efficacy profile of a therapeutic) information about a particular patient may affect dosage used. Continuous daily dosing may not be required; a therapeutic regimen may require cycles, during which time a drug is not administered, or therapy may be provided on an as-needed basis during periods of acute disease worsening. Dosage escalation may or may not be required; a therapeutic regimen may require reduction in medication dosage. Toxicity and therapeutic efficacy of the active ingredients described herein can be determined by standard pharmaceutical procedures in vitro, in cell cultures or experimental animals. The data obtained from these in-vitro and cell culture assays and animal studies can be used in formulating a range of dosage for use in human. The dosage may vary depending upon the dosage form employed and the route of administration utilized. The exact formulation, route of administration, and dosage can be chosen by the individual physician in view of the patient's condition (See, e.g., Fingl, E. et al. (1975), "The Pharmacological Basis of Therapeutics," Ch. 1, p. 1). Depending on the severity and responsiveness of the condition to be treated, dosing can be of a single or a plurality of administrations, with course of treatment lasting from several days to several weeks, or until cure is effected or diminution of the disease state is achieved.

The present invention further provides, in another aspect, a dosage unit comprising or consisting of any one of the pharmaceutical compositions described above.

In certain embodiments, the dosage unit comprises the pharmaceutical composition described above. In certain embodiments, the dosage unit consists of the pharmaceutical composition described above. In certain embodiments, the dosage unit is formulated as a gel, a powder or a spray. In certain embodiments, the dosage unit is formulated as a gel. In certain embodiments, the dosage unit is formulated as a powder. In certain embodiments, the dosage unit is formulated as a spray.

In certain embodiments, the dosage unit comprises or consists of a daily dose, a daily sub-dose or an appropriate fraction thereof, of the active ingredients. In certain embodiments, the dosage unit comprises a daily dose of the active ingredients. In certain embodiments, the dosage unit comprises a daily sub-dose of the active ingredients. In certain embodiments, the dosage unit consists of a daily dose of the active ingredients. In certain embodiments, the dosage unit consists of a daily sub-dose of the active ingredients. Unless otherwise indicated, the term "active ingredients" refers to opioids and N-acylethanolamines.

The phrase "a condition amenable to prevention or treatment by at least one opioid" as used herein generally relates to any adverse health condition, disease or disorder, such as pain, depression, respiratory difficulties, coughs and diarrhea, which is prevented or ameliorated or which at least one of its symptoms is prevented or ameliorated, by treatment with at least one opioid.

Also disclosed herein is that the condition amenable to prevention or treatment by at least one opioid is selected from the group consisting of pain, depression, respiratory difficulties, shortness of breath, coughs, irritable bowel syndrome and diarrhea.

The term "treating" as used herein, includes, but is not limited to, any one or more of the following: abrogating, ameliorating, inhibiting, attenuating, blocking, suppressing, reducing, delaying, halting, alleviating or preventing one or more symptoms or side effects of the diseases or conditions of the invention.

The term "acute" refers to a condition with a relatively short, severe course. The term "chronic" as used herein means that the length of time of the diseases or conditions of the invention can be weeks, months, or possibly years. The intensity of the diseases or conditions can differentiate according to various conditions such as patient age, temperature, season, type of disease, etc.

Neuropathic pain is a localized sensation of unpleasant discomfort caused by damage or disease that affects the somatosensory system. The International Association for the Study of Pain (IASP) widely used definition of pain states: "Pain is an unpleasant sensory and emotional experience associated with actual or potential tissue damage, or described in terms of such damage". Therefore, the term "pain", as used herein, means an unpleasant sensory and emotional experience associated with actual or potential tissue damage, or described in terms of such damage. Neuropathic pain may be associated with abnormal sensations called dysesthesia, and pain from normally non-painful stimuli (allodynia). It may have continuous and/or episodic (paroxysmal) components. The latter resemble stabbings or electric shocks. Common qualities include burning or coldness, "pins and needles" sensations, numbness and itching. Nociceptive pain, by contrast, is more commonly described as aching. Central neuropathic pain is found in spinal cord injury, multiple sclerosis, and some strokes. Aside from diabetes and other metabolic conditions, the common causes of painful peripheral neuropathies are herpes zoster infection, HIV-related neuropathies, nutritional deficiencies, toxins, remote manifestations of malignancies, immune mediated disorders and physical trauma to a nerve trunk. Neuropathic pain is common in cancer as a direct result of cancer on peripheral nerves (e.g., compression by a tumor), or as a side effect of chemotherapy (chemotherapy-induced peripheral neuropathy), radiation injury or surgery.

The present invention further provides, in another aspect, a pharmaceutical composition or a dosage unit as described above, for use in a method for preventing or treating at least one side-effect associated with intake of oxycodone or a salt thereof.

The term "intake" in the present invention is used in its broadest sense, and generally means the administration, use, consumption or ingestion of a substance, for example an opioid, to or by a subject, by any route. For example, the term "intake" is used to describe administration of opioids to human subjects, for example by oral administration, by transdermal administration, or by injection. The term "intake" in the present invention further encompasses the substances, or quantities thereof, taken in and used by the body; this refers to all routes by which opioids enter the body, including by mouth and parenteral administration.

In certain embodiments, the side-effect is irritation. In certain embodiments, the side-effect is confusion, uncontrolled movement and/or disorientation. Each possibility represents a separate embodiment of the invention. In certain embodiments, the side-effect is confusion.

In certain embodiments, the side-effect is associated with intake of oxycodone or a salt thereof.

Also disclosed herein is that the therapeutic potency of the pharmaceutical composition is increased compared to the therapeutic potency of the same pharmaceutical composition without the N-acylethanolamine. Also disclosed herein is that the required therapeutic dosage of the opioid in the pharmaceutical composition is decreased compared to the required therapeutic dosage of the opioid of the same pharmaceutical composition without the N-acylethanolamine. In certain embodiments, at least one of the side-effects of intake of at least one opioid is ameliorated compared to the same side-effect of intake of the same opioid of the same pharmaceutical composition without the N-acylethanolamine. As disclosed herein, the therapeutic window of the opioid is expended compared to the therapeutic window of the opioid of the same pharmaceutical composition without the N-acylethanolamine. As disclosed herein, the therapeutic effect of the pharmaceutical composition decreases slower than the therapeutic effect of the same pharmaceutical composition without the N-acylethanolamine.

Also disclosed herein is that the therapeutic potency of the pharmaceutical composition is increased compared to the same pharmaceutical composition without the N-acylethanolamine. Disclosed herein is that the required therapeutic dosage of the opioid in the pharmaceutical composition is decreased compared to the required therapeutic dosage of the opioid in a similar pharmaceutical composition without the N-acylethanolamine. In certain embodiments, at least one of the side-effects of the opioid in the pharmaceutical composition is reduced by the use of the pharmaceutical composition compared to the same side-effect while using the same pharmaceutical composition without the N-acylethanolamine. Also disclosed is that the therapeutic window of the opioid in the pharmaceutical composition is expended compared to the therapeutic window of the opioid in a similar pharmaceutical composition without the N-acylethanolamine.

Disclosed herein is that the N-acylethanolamine increases the therapeutic potency of the opioid compared to the same pharmaceutical composition without the N-acylethanolamine. Disclosed herein is that the N-acylethanolamine decreases the required therapeutic dosage of the opioid compared to the same pharmaceutical composition without the N-acylethanolamine. In certain embodiments, the N-acylethanolamine reduces at least one of the side-effects of the opioid compared to the same pharmaceutical composition without the N-acylethanolamine. As disclosed herein, the N-acylethanolamine expends the therapeutic window of the opioid compared to the same pharmaceutical composition without the N-acylethanolamine. As disclosed herein, the PEA or salt thereof increases the therapeutic potency of the oxycodone or salt thereof compared to the same pharmaceutical composition without the PEA or salt thereof. As disclosed herein, the PEA or salt thereof decreases the required therapeutic dosage of the oxycodone or salt thereof compared to the same pharmaceutical composition without the PEA or salt thereof. In certain embodiments, the PEA or salt thereof reduces at least one of the side-effects of the oxycodone or salt thereof compared to the same pharmaceutical composition without the PEA or salt thereof. As disclosed herein, the PEA or salt thereof expends the therapeutic window of the oxycodone or salt thereof compared to the same pharmaceutical composition without the PEA or salt thereof.

The phrase "N-acylethanolamine increases the therapeutic potency of the opioid" as used herein refers to the significantly improved therapeutic effect of the opioid when administered with an N-acylethanolamine, compared to the therapeutic effect of the opioid when administered without the N-acylethanolamine.

The phrase "N-acylethanolamine decreases the required therapeutic dosage of the opioid" as used herein refers to the significantly lower dosage required to achieve a certain therapeutic effect of the opioid when administered with an N-acylethanolamine, compared to the N-acylethanolamine dosage required to achieve the same therapeutic effect when the opioid is administered without the N-acylethanolamine.

The phrase "N-acylethanolamine reduces at least one of the side effects of the opioid" as used herein refers to the significantly lower severity of at least one of the side effects of the opioid when the opioid is administered with an N-acylethanolamine, compared to the severity of the same side effect when the opioid is administered without the N-acylethanolamine.

The phrase "N-acylethanolamine prolongs the therapeutic window of the opioid" as used herein refers to the significantly longer period in which the opioid has a therapeutic effect when administered with an N-acylethanolamine, compared to the period in which the opioid has a therapeutic effect when administered without the N-acylethanolamine.

The present invention further provides, in another aspect, a composition according to claim 1 for use in a method for preventing or treating at least one side-effect associated with intake of opioid in a human subject in need thereof, the method comprising the step of administering to the subject a pharmaceutical composition comprising a therapeutically-effective amount of a mixture of at least one opioid or a salt thereof and at least one N-acylethanolamine or a salt thereof, thereby preventing or treating the at least one side-effect.

The present invention further provides, in another aspect, a composition according to claim 1 for use in a method for preventing or treating at least one side-effect associated with intake of opioid in a human subject in need thereof, the method comprising the step of administering to the subject a pharmaceutical composition comprising a therapeutically-effective amount of a mixture of at least one opioid or a salt thereof and at least one N-acylethanolamine or a salt thereof, wherein the molar ratio between the opioid and the N-acylethanolamine is between about 1:1 to about 1:100, thereby preventing or treating the at least one side-effect.

In certain embodiments of the compositions for use in methods described above, the administration of the opioid and the N-acylethanolamine is repeated. In certain embodiments of the compositions for use described above, the administration of the opioid and the N-acylethanolamine is repeated three times a day. In certain embodiments of the compositions for use described above, the administration of the opioid and the N-acylethanolamine is repeated twice a day. In certain embodiments of the compositions for use described above, the administration of the opioid and the N-acylethanolamine is repeated once a day. In certain embodiments of the compositions for use described above, the administration of the opioid and the N-acylethanolamine is repeated once every two days. In certain embodiments of the compositions for use described above, the administration of the opioid and the N-acylethanolamine is repeated once every three days.

The term "about" as used herein in relation to a value, a plurality of values or a range of values defined by a lowest and highest values means a value which is 10% lower and/or higher than the corresponding value, plurality of values or range of values. For example, the phrase "about 1" means "0.9 to 1.1", the phrase "about 1 or 2" means "0.9 to 1.1 or 1.8 to 2.2", and the phrase "about 1 to about 2" means "0.9 to 2.2".

While the present invention has been described with reference to certain embodiments, it will be understood by those skilled in the art that various changes may be made and equivalents may be substituted without departing from the scope of the present invention. In addition, many modifications may be made to adapt a particular situation or material to the teachings of the present invention without departing from its scope. Therefore, it is intended that the present invention not be limited to the particular embodiment disclosed, but that the present invention will include all embodiments falling within the scope of the appended claims.

The following examples are presented in order to more fully illustrate some embodiments of the invention. They should, in no way be construed, however, as limiting the broad scope of the invention.

### EXAMPLES

### Materials and Methods

**Oxycodone formulation for 10 and 5 mg/kg dose levels** - Oxycodone was prepared at a 1 mg/ml concentration by dissolving 10 mg Oxycodone in 10 ml of ethanol: cremophor: saline mixture at a 1:1:18 ratio. To prepare 0.5 mg/ml Oxycodone, 5 ml from the previous mixture was diluted with 5 ml of ethanol: cremophor: saline mixture at a 1:1:18 ratio. The formulations were prepared twice, once for the open field test and again for the tail pinch test.

**PEA formulation for 25 mg/kg dose level** - PEA was prepared at a 5 mg/ml concentration by dissolving 30 mg PEA in 6 ml of ethanol: cremophor: saline mixture at a 1:1:18 ratio. To prepare 2.5 mg/ml PEA, 4 ml from the previous mixture were diluted with 4 ml of ethanol: cremophor: saline mixture at a 1:1:18 ratio. The formulations were prepared twice, once for the open field test and again for the tail pinch test.

**Animals** - Mice, strain ICR, male, 8 weeks of age at study initiation. The average animal body weight at study initiation was in the range of 31.97 ± 1.61 g. The minimum and maximum weight in each group did not exceed ± 20 % of group mean weight. Animals were randomly allocated to individual cages on the day of reception. Animals were acclimated for seven to nine days.

**Table 1 - Group allocation**

| **Group** | **Test item (mg/kg)** | **N** | **Dose Volume (ml/kg)** |
|---|---|---|---|
| 1M | Vehicle | 6 | 10 |
| 6M | Oxycodone (10) | 6 | |
| 7M | Oxycodone (5) | 6 | |
| 8M | PEA (25) +Oxycodone (10) | 6 | |
| 9M | PEA (25) +oxycodone (5) | 7 | |

Test item was administered IP at a dose volume of 10 ml/kg according to the doses in Table 1. Dosing was performed 15 minutes before each of the behavioral tests.

**Table 2 -Murine dosages**

| **Group** | **Test item** | **Murine dose** | **Drug molar ratio *** |
|---|---|---|---|
| 6M | Oxycodone | 10 mg/kg | - |
| 7M | Oxycodone | 5 mg/kg | - |
| 8M | Oxycodone | 10 mg/kg | 1 |
| | PEA | 25 mg/kg | 2.5 |
| 9M | Oxycodone | 5 mg/kg | 1 |
| | PEA | 25 mg/kg | 5 |

| | | | |
|---|---|---|---|
| * The M.W of oxycodone is 315.364 g/mol, and the M.W of PEA is 299.50 g/mol. | | | |

**Open Field (OF) tests** were performed as follows - Fifteen minutes after test item/vehicle administration, mice were placed at the center of an open field box (43x43x40 cm) between 9 AM and 5 PM. On each side of the open field box, two frames placed at 2 and 5 cm height with 16 photocell beams per side ensure movement detection. The computer defined grid lines that divided the open field into two compartments: margin and center. Several variables were recorded during a 15 minute session of spontaneous activity including: time spent moving, traveled distance, time spent and number of visits to the central compartment.

**Tail pinch tests** were performed as follows, according to the modified Haffner's method (as depicted in Takagi et al., Jpn. J. Pharmacol., 1966, 16, Pages 287-294) - Mice were pretested by pinching their tail base with an artery clip (1.5 mm width, constant force), and only the mice that show a nociceptive response such as biting the clip or vocalizing within 2 sec were used for experiments. When the mice did not show the above-mentioned behaviors up to 6 sec after pinching, the antinociceptive effect was regarded as positive. To prevent tissue damage, the pressure stimuli was not applied for more than 10 sec. After drug treatments, the nociceptive response in the tail-pinch test was measured at varying intervals.

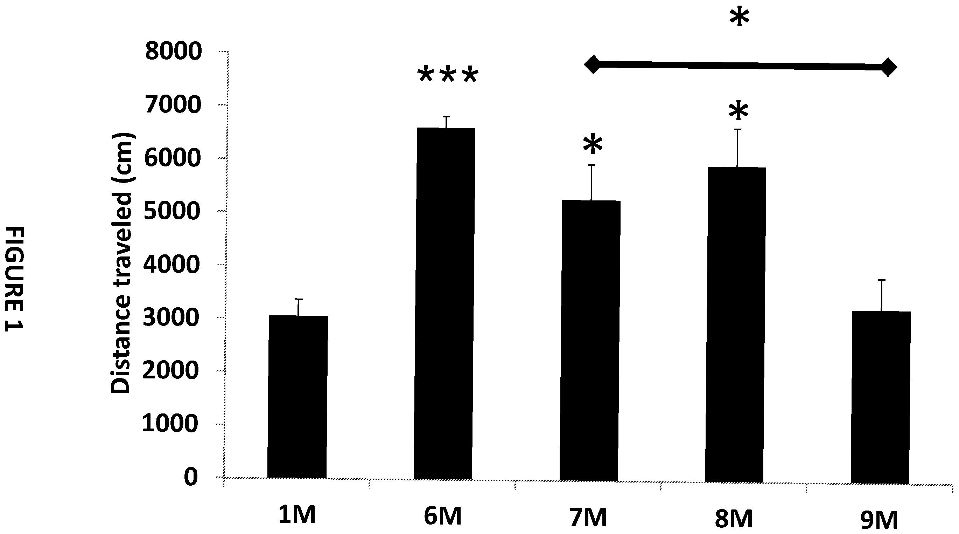

**Statistical analysis** - Numerical results were given as means ± standard error of the mean. The results were subjected either to a T-test or to two-way ANOVA, followed by Bonferroni post-hoc contrast analysis between the groups where applicable. A probability of less than 5% (p < 0.05) was regarded as significant. P values lower than 0.05 are indicated in Figures 1-3 as (*), P values lower than 0.01 are indicated in Figures 1-3 as (**), and P values lower than 0.001 are indicated in Figures 1-3 as (^{∗∗∗}),

### Example 1. Distance travelled.

Total distance traveled, associated with the irritation level of a subject, was evaluated during a 15 minute session in the open field test. As illustrated in Figure 1, oxycodone significantly increased the total distance traveled in a dose-dependent manner (see 6M, 8M compared to 1M). Total distance traveled was found to be significantly higher in oxycodone 10 mg/kg (6M, P<0.001) and oxycodone 10mg/kg combined with PEA 25 mg/kg (8M, P<0.05) compared to vehicle control group (1M) as displayed in Figure 1. PEA (25 mg/kg) in combination with low (5 mg/kg) or high (10 mg/kg) dose oxycodone showed a significant decrease in total distance travelled (see 9M, 7M, respectively). The effect of PEA is most notable when comparing groups 6M and 8M, and in comparing group 7M to group 1M and group 9M. The ability of PEA to significantly lower or prevent the oxycodone-related increase in total distance traveled is equivalent to preventing or minimizing adverse side-effects commonly associated with opioid uptake in humans, such as irritation (Crane JH et al., Int. J. Clin. Pharm., 2011, Vol. 33(5), pages 733-736; Tanaka R et al., Am. J. Hosp. Palliat. Care, 2016).

### Example 2. Animal velocity.

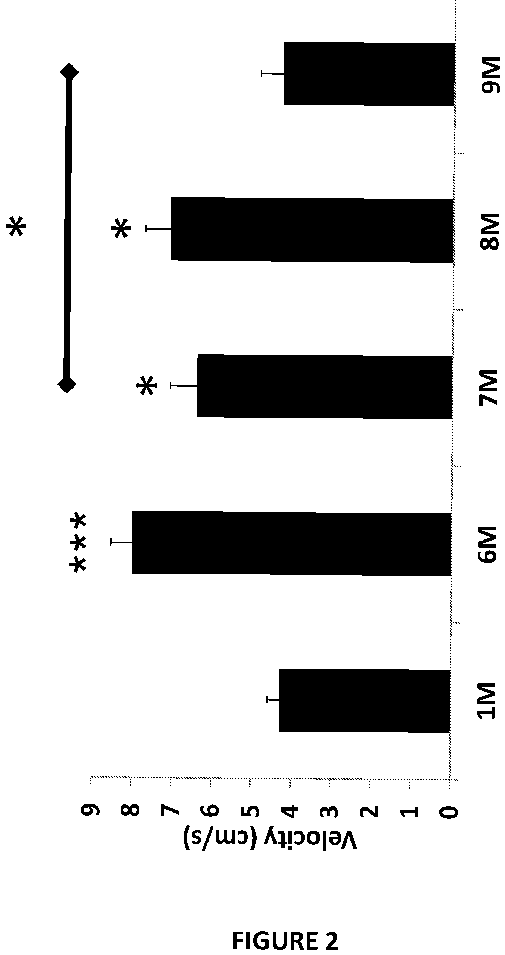

Average animal velocity, associated with uncontrolled movement and confusion, was calculated by dividing the total distance traveled (cm) by each animal by the total moving time (sec) during a 15 minute session in an open field test. As illustrated in Figure 2, oxycodone significantly increased the velocity of the animals in a dose-dependent manner (see 6M, 8M compared to 1M). The velocity was found to be significantly higher in oxycodone 10 mg/kg (6M, P<0.001) and oxycodone 10 mg/kg combined with PEA 25 mg/kg (8M, P<0.05) compared to vehicle control group (1M) as displayed in Figure 2. PEA (25 mg/kg) in combination with low (5 mg/kg) or high (10 mg/kg) dose oxycodone showed a significant decrease in velocity (see 9M, 7M, respectively). The effect of PEA is most notable when comparing groups 6M and 8M, and in comparing group 7M to group 1M and group 9M. As in Example 1, the ability of PEA to significantly lower or prevent the oxycodone-related increase in average animal velocity is equivalent to preventing or minimizing adverse side-effects commonly associated with opioid uptake in human, such as confusion and/or disorientation (Metrik J. et al., J. Cogn. Psychother., 2011, pages 1-18).

### Example 3. Tail Pinch test.

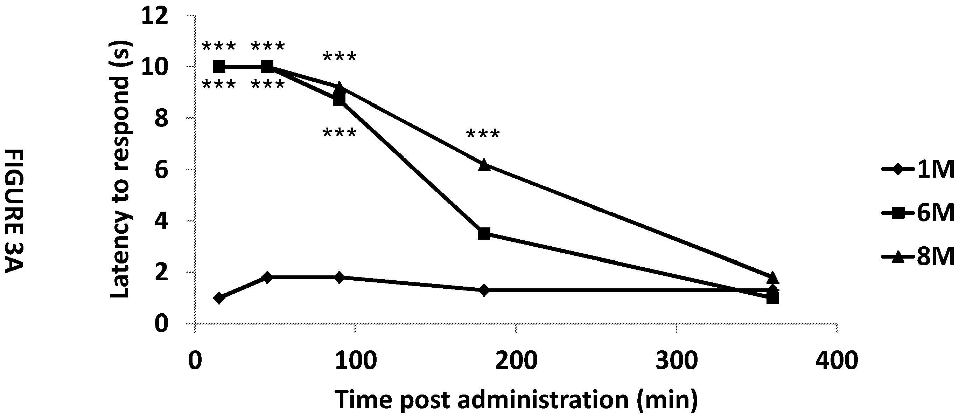

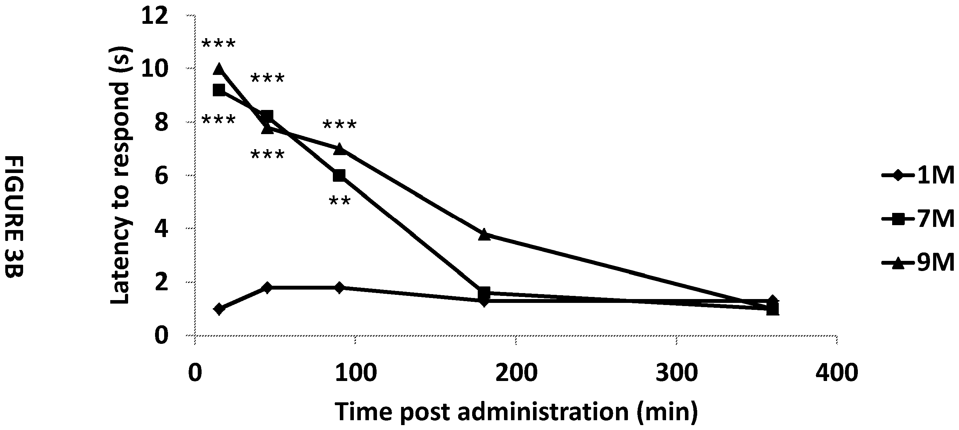

The tail pinch test was performed 15 minutes after the administration of the indicated test item. Pressure was applied to the base of the tail for no more than 10 seconds. As shown in Figures 3A, while a high dose of oxycodone provided a significant analgesic effect which began 15 minutes after administration and lasted at least 90 minutes (6M, P<0.001), a high dose of oxycodone combined with PEA provided a significant and extended analgesic effect which began 15 minutes after administration and lasted at least 180 minutes (8M, P<0.001), doubling the effective time of analgesia compared to the same treatment without PEA (6M). The same is true regarding the use of low-dose oxycodone. As shown in Figures 3B, while a low dose of oxycodone provided a significant analgesic effect which began 15 minutes after administration and lasted at least 90 minutes (7M, P<0.01), a low dose of oxycodone combined with PEA provided a more pronounced and significant analgesic effect which began 15 minutes after administration and lasted at least 90 minutes (9M, P<0.001). More, the addition of a PEA to a low dose of oxycodone (9M) extended the analgesic effect, or delayed its diminishing, beyond the 90 minutes mark and towards 180 minutes, in a manner that almost reached statistical significance.

From the data presented in Figure 3A and 3B it is evident that the combination of N-acylethanolamines and opioids is fast-acting, highly-potent and long-lasting in preventing or minimizing pain, compared to the analgesic effect offered by opioids alone.

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed functions may take a variety of alternative forms without departing from the invention.

## Claims

1. A pharmaceutical composition for use in a method for preventing or treating at least one side-effect associated with intake of an opioid, wherein the opioid is oxycodone or a salt thereof, wherein the at least one side-effect is selected from irritation, confusion, uncontrolled movement and/or disorientation, wherein the composition comprises a therapeutically-effective amount of a mixture of at least one opioid or a salt thereof and at least one N-acylethanolamine or a salt thereof, wherein the molar ratio between the opioid or the salt thereof and the N- acylethanolamine or the salt thereof is between about 1:1 to about 1:100, wherein the at least one opioid is oxycodone or a salt thereof, and wherein the N-acylethanolamine is palmitoylethanolamine (PEA) or a salt thereof.

2. The pharmaceutical composition for use of claim 1, wherein the molar ratio is
(a) between about 1:2 to about 1:80; or
(b) between about 1:2.5 to about 1:5

3. The pharmaceutical composition for use of claim 1 or 2, comprising
(a) about 1-100 mg opioid or a salt thereof;
(b) about 20 mg opioid or a salt thereof;
(c) about 35 mg opioid or a salt thereof;
(d) about 55 mg opioid or a salt thereof;
(e) about 70 mg opioid or a salt thereof; or
(f) about 90 mg opioid or a salt thereof.

4. The pharmaceutical composition for use of claim 1 or 2, comprising
(a) about 200- 1800 mg N-acylethanolamine or a salt thereof;
(b) about 250 mg N-acylethanolamine or a salt thereof;
(c) about 500 mg N-acylethanolamine or a salt thereof;
(d) about 750 mg N-acylethanolamine or a salt thereof;
(e) about 1000 mg N-acylethanolamine or a salt thereof; or
(f) about 1500 mg N-acylethanolamine or a salt thereof.

5. The pharmaceutical composition for use of claim 1, wherein
(a) the molar ratio between the oxycodone or the salt thereof and the PEA or the salt thereof is between about 1 :2.5 to about 1:5;
(b) the molar ratio between the oxycodone or the salt thereof and the PEA or the salt thereof is about 1 :2.5; or
(c) the molar ratio between the oxycodone or the salt thereof and the PEA or the salt thereof is about 1:5.

6. The pharmaceutical composition for use of claim 1, wherein the mixture comprises
(a) about 1-100 mg oxycodone or a salt thereof or about 200-1800 mg PEA or a salt thereof; or
(b) about 20 mg, about 35 mg, about 55 mg, about 70 mg or about 90 mg oxycodone or a salt thereof or about 250 mg, about 500 mg, about 750 mg, about 1000 mg or about 1500 mg PEA or a salt thereof.

7. The pharmaceutical composition for use of any one of claims 1 to 6, wherein the pharmaceutical composition is formulated for systemic administration

8. The pharmaceutical composition for use of claim 7, wherein the pharmaceutical composition is formulated for oral, oral mucosal, nasal, sublingual, topical, transdermal, rectal, parenteral, intravenous, intramuscular, subcutaneous, intrathecal, inhalational or vaginal administration.

9. The pharmaceutical composition for use of claim 8, wherein the pharmaceutical composition is formulated for oral, oral mucosal, nasal or sublingual administration.

10. A dosage unit comprising or consisting of the pharmaceutical composition for use of any one of claims 1 to 9.

11. The pharmaceutical composition for use according to any one of claims 1 to 9, wherein at least one of the side-effects of intake of at least one opioid is ameliorated compared to the same side-effect of intake of the same opioid of the same pharmaceutical composition without the N-acylethanolamine.

12. The composition for use according to any one of claims 1 to 9 or 11, wherein the administration of the opioid and the N-acylethanolamine is repeated.

## Patentansprüche

1. Pharmazeutische Zusammensetzung zur Verwendung in einem Verfahren zur Vorbeugung oder Behandlung von zumindest einer Nebenwirkung im Zusammenhang mit Einnahme eines Opioids, wobei das Opioid Oxycodon oder ein Salz davon ist, wobei die zumindest eine Nebenwirkung ausgewählt ist aus Reizung, Verwirrtheit, unkontrollierter Bewegung und/oder Desorientierung, wobei die Zusammensetzung eine therapeutisch wirksame Menge einer Mischung aus zumindest einem Opioid oder einem Salz davon und zumindest einem N-Acylethanolamin oder einem Salz davon umfasst, wobei das Molverhältnis zwischen dem Opioid oder dem Salz davon und dem N-Acylethanolamin oder dem Salz davon zwischen etwa 1:1 und etwa 1: 100 ist, wobei das zumindest eine Opioid Oxycodon oder ein Salz davon ist und wobei das N-Acylethanolamin Palmitoylethanolamin (PEA) oder ein Salz davon ist.

2. Pharmazeutische Zusammensetzung zur Verwendung nach Anspruch 1, wobei das Molverhältnis wie folgt ist
(a) zwischen etwa 1:2 und etwa 1:80; oder
(b) zwischen etwa 1:2,5 und etwa 1:5.

3. Pharmazeutische Zusammensetzung zur Verwendung nach Anspruch 1 oder 2, umfassend
(a) etwa 1-100 mg Opioid oder eines Salzes davon;
(b) etwa 20 mg Opioid oder eines Salzes davon;
(c) etwa 35 mg Opioid oder eines Salzes davon;
(d) etwa 55 mg Opioid oder eines Salzes davon;
(e) etwa 70 mg Opioid oder eines Salzes davon; oder
(f) etwa 90 mg Opioid oder eines Salzes davon.

4. Pharmazeutische Zusammensetzung zur Verwendung nach Anspruch 1 oder 2, umfassend
(a) etwa 200-1800 mg N-Acylethanolamin oder eines Salzes davon;
(b) etwa 250 mg N-Acylethanolamin oder eines Salzes davon;
(c) etwa 500 mg N-Acylethanolamin oder eines Salzes davon;
(d) etwa 750 mg N-Acylethanolamin oder eines Salzes davon;
(e) etwa 1000 mg N-Acylethanolamin oder eines Salzes davon; oder
(f) etwa 1500 mg N-Acylethanolamin oder eines Salzes davon.

5. Pharmazeutische Zusammensetzung zur Verwendung nach Anspruch 1, wobei
(a) das Molverhältnis zwischen dem Oxycodon oder dem Salz davon und dem PEA oder dem Salz davon zwischen etwa 1:2,5 und etwa 1:5 ist;
(b) das Molverhältnis zwischen dem Oxycodon oder dem Salz davon und dem PEA oder dem Salz davon etwa 1:2,5 ist; oder
(c) das Molverhältnis zwischen dem Oxycodon oder dem Salz davon und dem PEA oder dem Salz davon etwa 1:5 ist.

6. Pharmazeutische Zusammensetzung zur Verwendung nach Anspruch 1, wobei die Mischung Folgendes umfasst
(a) etwa 1-100 mg Oxycodon oder eines Salzes davon oder etwa 200-1800 mg PEA oder eines Salzes davon; oder
(b) etwa 20 mg, etwa 35 mg, etwa 55 mg, etwa 70 mg oder etwa 90 mg Oxycodon oder eines Salzes davon oder etwa 250 mg, etwa 500 mg, etwa 750 mg, etwa 1000 mg oder etwa 1500 mg PEA oder eines Salzes davon.

7. Pharmazeutische Zusammensetzung zur Verwendung nach einem der Ansprüche 1 bis 6, wobei die pharmazeutische Zusammensetzung zur systemischen Verabreichung formuliert ist.

8. Pharmazeutische Zusammensetzung zur Verwendung nach Anspruch 7, wobei die pharmazeutische Zusammensetzung zur oralen, oral-mukosalen, nasalen, sublingualen, topischen, transdermalen, rektalen, parenteralen, intravenösen, intramuskulären, subkutanen, intrathekalen, inhalativen oder vaginalen Verabreichung formuliert ist.

9. Pharmazeutische Zusammensetzung zur Verwendung nach Anspruch 8, wobei die pharmazeutische Zusammensetzung zur oralen, oral-mukosalen, nasalen oder sublingualen Verabreichung formuliert ist.

10. Dosierungseinheit, umfassend die pharmazeutische Zusammensetzung zur Verwendung nach einem der Ansprüche 1 bis 9 oder bestehend daraus.

11. Pharmazeutische Zusammensetzung zur Verwendung nach einem der Ansprüche 1 bis 9, wobei zumindest eine der Nebenwirkungen der Einnahme von zumindest einem Opioid verglichen mit der gleichen Nebenwirkung der Einnahme des gleichen Opioids der gleichen pharmazeutischen Zusammensetzung ohne das N-Acylethanolamin gelindert ist.

12. Zusammensetzung zur Verwendung nach einem der Ansprüche 1 bis 9 oder 11, wobei die Verabreichung des Opioids und des N-Acylethanolamins wiederholt wird.

## Revendications

1. Composition pharmaceutique pour l'utilisation dans une méthode de prévention ou de traitement d'au moins un effet secondaire associé à la prise d'un opioïde, dans laquelle l'opioïde est l'oxycodone ou un sel de celle-ci, au moins un effet secondaire étant choisi parmi irritation, confusion, mouvement incontrôlé et/ou désorientation, la composition comprenant une quantité thérapeutiquement efficace d'un mélange d'au moins un opioïde ou un sel de celui-ci et d'au moins une N-acylétanolamine ou un sel de celle-ci, le rapport molaire entre l'opioïde ou le sel de celui-ci et la N- l'acylétanolamine ou le sel de celle-ci se situant entre environ 1:1 et environ 1:100, au moins un opioïde étant l'oxycodone ou un sel de celle-ci, et la N-acylétanolamine étant la palmitoyléthanolamine (PEA) ou un sel de celle-ci.

2. Composition pharmaceutique pour l'utilisation de la revendication 1, dans laquelle le rapport molaire est
(a) entre environ 1:2 jusqu'à environ 1:80 ; ou
(b) entre environ 1:2,5 jusqu'à environ 1:5.

3. Composition pharmaceutique pour l'utilisation de la revendication 1 ou 2, comprenant
(a) environ 1 à 100 mg d'opioïde ou un sel de celui-ci ;
(b) environ 20 mg d'opioïde ou un sel de celui-ci ;
(c) environ 35 mg d'opioïde ou un sel de celui-ci ;
(d) environ 55 mg d'opioïde ou un sel de celui-ci ;
(e) environ 70 mg d'opioïde ou un sel de celui-ci ; ou
(f) environ 90 mg d'opioïde ou un sel de celui-ci.

4. Composition pharmaceutique pour l'utilisation de la revendication 1 ou 2, comprenant
(a) environ 200 à 1800 mg de N-acyléthanolamine ou un sel de celle-ci ;
(b) environ 250 mg de N-acyléthanolamine ou un sel de celle-ci ;
(c) environ 500 mg de N-acyléthanolamine ou un sel de celle-ci ;
(d) environ 750 mg de N-acyléthanolamine ou un sel de celle-ci ;
(e) environ 1000 mg de N-acyléthanolamine ou un sel de celle-ci ; ou
(f) environ 1500 mg de N-acyléthanolamine ou un sel de celle-ci.

5. Composition pharmaceutique pour l'utilisation de la revendication 1, dans laquelle
(a) le rapport molaire entre l'oxycodone ou le sel de celle-ci et la PEA ou le sel de celle-ci est compris entre environ 1:2,5 et environ 1:5 ;
(b) le rapport molaire entre l'oxycodone ou le sel de celle-ci et la PEA ou le sel de celle-ci est d'environ 1:2,5 ; ou
(c) le rapport molaire entre l'oxycodone ou le sel de celle-ci et la PEA ou le sel de celle-ci est d'environ 1:5.

6. Composition pharmaceutique pour l'utilisation de la revendication 1, dans laquelle le mélange comprend
(a) environ 1 à 100 mg d'oxycodone ou un sel de celle-ci ou environ 200 à 1800 mg de PEA ou un sel de celle-ci ; ou
(b) environ 20 mg, environ 35 mg, environ 55 mg, environ 70 mg ou environ 90 mg d'oxycodone ou un sel de celle-ci ou environ 250 mg, environ 500 mg, environ 750 mg, environ 1000 mg ou environ 1500 mg de PEA ou un sel de celle-ci.

7. Composition pharmaceutique pour l'utilisation de l'une quelconque des revendications 1 à 6, la composition pharmaceutique étant formulée pour l'administration systémique.

8. Composition pharmaceutique pour l'utilisation de la revendication 7, la composition pharmaceutique étant formulée pour l'administration orale, par la muqueuse buccale, nasale, sublinguale, topique, transdermique, rectale, parentérale, intraveineuse, intramusculaire, sous-cutanée, intrathécale, par inhalation ou vaginale.

9. Composition pharmaceutique pour l'utilisation de la revendication 8, la composition pharmaceutique étant formulée pour l'administration orale, par la muqueuse buccale, nasale ou sublinguale.

10. Unité posologique comprenant ou constituée de la composition pharmaceutique pour l'utilisation de l'une quelconque des revendications 1 à 9.

11. Composition pharmaceutique pour l'utilisation selon l'une quelconque des revendications 1 à 9, au moins un des effets secondaires de la prise d'au moins un opioïde étant amélioré par rapport au même effet secondaire de la prise du même opioïde de la même composition pharmaceutique sans la N- acyléthanolamine.

12. Composition pour l'utilisation selon l'une quelconque des revendications 1 à 9 ou 11, l'administration de l'opioïde et de la N-acyléthanolamine étant répétée.
